# EUROPÄISCHE PATENTSCHRIFT

(11) **EP 2 095 281 B1**
(45) Veröffentlichungstag und Bekanntmachung des Hinweises auf die Patenterteilung: **04.05.2016**
(21) Anmeldenummer: 07726171.7
(22) Anmeldetag: 28.06.2007
(51) Int. Cl.: A61B 5/00, G06F 19/00, A61B 5/08, A61M 16/00, G07C 9/00

(54) **MEDIZINISCHES SYSTEM**
MEDICAL SYSTEM
SYSTÈME MÉDICAL

(30) Priorität: 01.12.2006 DE 102006056723
(43) Veröffentlichungstag der Anmeldung: 02.09.2009
(73) Patentinhaber: Drägerwerk AG & Co. KGaA, 23558 Lübeck (DE)
(72) Erfinder: SCHERMEIER, Olaf, 23560 Lübeck (DE); WOTHA, Gerd, 23626 Warnsdorf (DE); OTTO, Andreas, 22941 Bargteheide (DE)
(86) Internationale Anmeldenummer: PCT/EP2007/005738
(87) Internationale Veröffentlichungsnummer: WO 2008/064725

(56) Entgegenhaltungen:
- EP-A- 1 731 089
- EP-A- 1 731 188
- WO-A-2004/079554
- WO-A-2005/082452
- WO-A-2005/112744
- WO-A-2007/059810
- US-A1- 2004 039 263
- US-A1- 2006 026 205
- YANG XIAO, XUEMIN SHEN, BO SUN, LIN CAI: "Security and Privacy in RFID and Applications in Telemedicine" IEEE COMMUNICATIONS MAGAZINE, April 2006 (2006-04), Seiten 64-72, XP002468207

## Beschreibung

Die vorliegende Erfindung betrifft ein medizinisches System mit einer Überwachungsstation und mit wenigstens einem medizinischen Arbeitsplatz, wobei der wenigstens eine medizinische Arbeitsplatz ausgebildet ist, physiologische Daten eines Patienten zu erfassen, und wobei der wenigstens eine medizinische Arbeitsplatz mit der Überwachungsstation wirkverbunden ist und dazu ausgebildet ist, ein die physiologischen Daten repräsentierendes Datensignal zu erzeugen und an die Überwachungsstation zu senden, wobei die Überwachungsstation ausgebildet ist, das Datensignal zu empfangen, und einen Speicher für wenigstens einen Patientendaten repräsentierenden Patientendatensatz und wenigstens eine Anzeigeeinheit zum Anzeigen der physiologischen Daten und der Patientendaten aufweist, und das medizinische System ausgebildet ist, die Patientendaten und den die physiologischen Daten einander zuzuordnen, wobei der wenigstens eine medizinische Arbeitsplatz eine Respirationsvorrichtung mit einem Anschluss für einen Respirationsschlauch aufweist und der medizinische Arbeitsplatz eine Radiofrequenzerfassungsvorrichtung aufweist, welche ausgebildet ist, eine Radiofrequenzmarkierung mit einer Markierungsinformation zu erfassen und ein Markierungssignal zu erzeugen, welches die Markierungsinformation repräsentiert, und das medizinische System ausgebildet ist, die physiologischen Daten und die in der erfassten Markierungsinformation enthaltenen Patientendaten in Abhängigkeit des Markierungssignals einander zuzuordnen, sowie ein entsprechendes Verfahren zum Zuordnen von Patientendaten zu physiologischen Daten.

Ein medizinisches System mit den Merkmalen des Oberbegriffs des Patentanspruchs 1 ist aus WO 2004/079554 A2 bekannt. Dabei werden auch Markierungsinformationen von einem medizinischen Arbeitsgerät erfasst und Patientendaten zugeordnet.

In dem Artikel "Security and Privacy in RFID and Applications in Telemedicine" von X. Yang et al., IEEE communications magazine, April 2006 (2006-04), Seiten 64 - 72, beschreibt Überwachungssysteme, in denen Patienten jeweils ein RFID-Transmitter zugeordnet wird, der auch mit Mikrosensoren zur Erfassung von physiologischen Daten (z.B. EKG-Daten, Körpertemperatur etc.) ausgestattet sein kann. In dem RFID-Transmitter sind auch jeweils Daten enthalten, die es erlauben, den jeweiligen Patienten zu identifizieren. Auf diese Weise können physiologische Daten, die jeweils einem bestimmten Patienten zugeordnet sind, in einer Zentraleinheit erfasst und ausgewertet werden.

Die der Erfindung zugrunde liegende Aufgabe ist es, ein medizinisches System anzugeben, welches ein verbessertes Zuordnen von Patientendaten und physiologischen Daten erlaubt, insbesondere wenn ein neuer Patient mit einem ihm schon zugeordneten Respirationsschlauch von einem anderen medizinischen Arbeitsplatz an den medizinischen Arbeitsplatz verlegt wird. Es ist ferner Aufgabe, ein entsprechendes Verfahren zum Zuordnen von Patientendaten und physiologischen Daten in einem medizinischen System anzugeben.

Zur Lösung dieser Aufgabe dienen das medizinische System mit den Merkmalen des Patentanspruchs 1 und das medizinische Verfahren zum Zuordnen von Patientendaten und physiologischen Daten mit den Merkmalen des Patentanspruchs 4. Vorteilhafte Ausführungen sind in den abhängigen Ansprüchen angegeben.

Das medizinische System besitzt einen medizinischen Arbeitsplatz mit einer Respirationsvorrichtung und einem Anschluss für einen Respirationsschlauch. Die Respirationsvorrichtung hat eine Radiofrequenzerfassungs-vorrichtung, welche ausgebildet ist, im Bereich des Anschlusses für den Respirationsschlauch eine mit einem Respirationsschlauch verbundene Radiofrequenzmarkierung mit Markierungsinformationen zu erfassen und ein Markierungssignal zu erzeugen, welches die Markierungsinformation repräsentiert. Das medizinische System ist ausgebildet, die physiologischen Daten mit einem Monitor zu erfassen und die Patientendaten, insbesondere einen dem Datensignal entsprechenden Datensatz und den Patientendatensatz, in Abhängigkeit des Markierungssignals einander zuzuordnen. Durch ein medizinisches System mit einer Respirationsvorrichtung und einer Radiofrequenzerfassungsvorrichtung kann vorteilhaft ein eindeutiges, sicheres Zuordnen von erfassten, physiologischen Daten zu Patientendaten erfolgen.

Das medizinische System umfasst auch wenigstens einen Respirationsschlauch mit einer Radiofrequenzmarkierung, wobei die Radiofrequenzmarkierung eine Markierungsinformation repräsentiert. Beispielsweise kann die Radiofrequenzmarkierung dazu den die Markierungsinformation repräsentierenden Markierungs- Datensatzes auslesbar vorrätig halten.

Die Radiofrequenzmarkierung einen Schreib-Lese-Speicher für den die Markierungsinformation repräsentierenden Markierungs- Datensatz auf, welcher als Markierungsinformation wenigstens Patientendaten enthält.

Auf diese Weise kann ein Respirationsschlauch persönliche Patientendaten vorrätig halten, so dass ein sicheres Zuordnen der Patientendaten zu den von dem Patienten erfassten physiologischen Daten möglich ist. Wenn ein Patient beispielsweise auf einer Intensivstation eines Krankenhauses von einem medizinischen Arbeitsplatz zu einem anderen medizinischen Arbeitsplatz transportiert wird, so kann der Patient den Respirationsschlauch mit den Patientendaten mitführen. Der Respirationsschlauch wird nach einem Transport des Patienten an einen anderen medizinischen Arbeitsplatz an dem anderen medizinischen Arbeitsplatz angeschlossen. Der andere medizinische Arbeitsplatz kann daraufhin die Radiofrequenzmarkierung erfassen, den Markierungsdatensatz auslesen und ein Markierungssignal erzeugen, welches die Pätientendaten repräsentiert, und nach einem Anschließen des Respirationsschlauches erfasste physiologische Daten des Patienten zusammen mit der Markierungsinformation an die Überwachungsstation senden. Auf diese Weise kann ein genaues Zuordnen der physiologischen Daten zu den Patientendaten erfolgen.

Dadurch kann der medizinische Arbeitsplatz vorteilhaft einen weiteren Betrieb, beispielsweise einen Respirationsvorgang, in Abhängigkeit von den ausgelesenen Erfassungsparametern und/oder den ausgelesenen Daten durchführen oder fortsetzen.

Beispielhafte Ausführungsformen für physiologische Daten sind ein Sauerstoffanteil oder ein Kohlendioxidanteil eines Respirations-Gasstromes oder ein Atemminutenvolumen.

Beispielhafte Ausführungsformen für eine Respirations-vorrichtung eines medizinischen Arbeitsplatzes sind eine Beatmungsvorrichtung oder ein Anästhesiebeatmungsgerät. Die Respirationsvorrichtung ist ausgebildet, eine Inspiration oder eine Expiration zu erzeugen. Dazu weist die Respirations-vorrichtung eine Ventilationsvorrichtung und einen mit dieser mindestens mittelbar verbundenen Anschluss für einen Respirationsschlauch auf. Die Respirationsvorrichtung weist bevorzugt wenigstens einen Gassensor auf und ist ausgebildet, mittels des Gassensors physiologische Daten, insbesondere Respirationsdaten eines an die Respirationsvorrichtung angeschlossenen Patienten zu erfassen. Der Gassensor ist beispielsweise ein Sauerstoffsensor oder ein Kohlendioxid-sensor, welche jeweils ausgebildet sind, einen Gasanteil, insbesondere Sauerstoff beziehungsweise Kohlendioxid, eines Atemgasstromes zu erfassen und ein den Gasanteil repräsentierendes Gasanteilsignal erzeugen. Die Respirationsvorrichtung kann weiter einen Atemminutenvolumensensor aufweisen, welcher ein Atemminutenvolumen des Atemgasstromes erfassen kann und ein das Atemminutenvolumen repräsentierendes Minutenvolumensignal erzeugen kann. Die Respirationsvorrichtung ist kann ausgebildet sein, einen dem Gasanteilsignal und/oder Minutenvolumensignal entsprechenden Datensatz zu erzeugen, welcher somit physiologische Daten des Patienten repräsentiert.

In einer bevorzugten Ausführungsform des medizinischen Systems ist wenigstens ein medizinischer Arbeitsplatz drahtlos mit der Überwachungsstation verbunden. Wenigstens ein weiterer medizinischer Arbeitsplatz kann drahtgebunden mit der Überwachungsstation verbunden sein. Beispielhafte Ausführungsformen für eine drahtlose Verbindung eines medizinischen Arbeitsplatzes mit der Überwachungsstation ist eine WLAN-Verbindung, eine Bluetooth-Verbindung, eine Funk- Verbindung, eine optische Verbindung, insbesondere eine Infrarot-Verbindung, beispielsweise eine IrDA-Verbindung (IrDA = Infrared Data Association). Dazu kann der medizinische Arbeitsplatz einen der drahtlosen Verbindungsart entsprechenden Sender aufweisen. Das medizinische System weist einen der drahtlosen Verbindungsart entsprechenden Empfänger auf, welcher mit der Überwachungsstation verbunden ist. Auf diese Weise kann eine Überwachungsstation zusammen mit wenigstens zwei medizinischen Arbeitsplätzen ein medizinisches System, umfassend ein Netzwerk mit medizinischen Arbeitsplätzen bilden.

Beispielhafte Ausführungsformen für ein medizinisches System mit einer schnurgebundenen Verbindung zwischen der Überwachungsstation und dem wenigstens einen medizinischen Arbeitsplatz sind eine LAN-Verbindung (LAN = Local Area Network), eine serielle Verbindung, beispielsweise eine USB-Verbindung, oder eine schnelle Verbindung, insbesondere eine Hochgeschwindigkeitsverbindung.

Die Radiofrequenzerfassungsvorrichtung kann ausgebildet sein, in einem Frequenzbereich von 30 Kilohertz bis 500 Kilohertz, in einem Frequenzbereich von 800 bis 950 Megahertz, oder in einem Frequenzbereich von 1 Gigahertz bis 3 Gigahertz zu arbeiten.

Die Radiofrequenzerfassungsvorrichtung kann ausgebildet sein, mit einer Frequenz von 125 Kilohertz oder mit einer Frequenz von 13,56 Megahertz zu arbeiten. Die Radiofrequenzerfassungsvorrichtung und die Radiofrequenzmarkierung können beispielsweise beim Übertragen eines die Markierungsinformation repräsentierenden Markierungssignals mit einem der folgenden Modulationsverfahren oder einer Kombination aus den folgenden Modulationsverfahren arbeiten:
- FM (FM = Frequenzmodulation);
- AM (AM = Amplitudenmodulation);
- FSK (FSK = Frequency Shift Keying);
- ASK (ASK = Amplitude Shift Keying);
- PSK (PSK = Phase Shift Keying).

Die Radiofrequenzerfassungsvorrichtung kann die Radiofrequenzmarkierung mittels Lastmodulation erfassen. Dabei kann die Radiofrequenzerfassungsvorrichtung die Radio-frequenzmarkierung mit Sendeenergie versorgen.

Beispielsweise kann die Radiofrequenzmarkierung dazu einen Energiespeicher aufweisen und die empfangene Sendeenergie speichern und zum Rücksenden eines die Markierungsinformation repräsentierenden Antwort-Sendesignals nutzen. Die Radiofrequenzmarkierung kann eine aktive Radiofrequenzmarkierung sein, welche eine Energiequelle zum Senden der Markierungs-information aufweist.

Der Schreib-Lese-Speicher kann Speicher zum Vorrätighalten von physiologischen Daten eine Größe von mindestens 50 Kilobyte, bevorzugt 500 Kilobyte.
Die Radiofrequenzmarkierung kann einen mit dem Schreib-Lesespeicher verbundenen Speichercontroller aufweisen. Dadurch kann vorteilhaft in einem medizinischen System ein richtiges Zuordnen von physiologischen Daten zu Patientendaten erfolgen.

Der medizinische Arbeitsplatz kann dazu ausgebildet sein, beim Verbinden eines Respirationsschlauches mit dem medizinischen Arbeitsplatz mittels der Radiofrequenz-erfassungsvorrichtung den auf der Radiofrequenzmarkierung abgespeicherten Datensatz wieder auszulesen und ein weiteres Erfassen von physiologischen Daten in Abhängigkeit von den ausgelesenen Parametern durchzuführen. Auf diese Weise können zum Betreiben eines medizinischen Arbeitsplatzes erforderliche Einstellungs- bzw. Geräteparameter, welche insbesondere individuell für einen Patienten erstellt worden sind, vorteilhaft auf der Radiofrequenzmarkierung des Respirationsschlauches abgespeichert werden. Wenn ein Patient zu einem anderen medizinischen Arbeitsplatz transportiert wird, welcher beispielsweise in einem anderen Behandlungsraum angeordnet ist, kann dort ein Betrieb des in der anderen medizinischen Arbeitsplatzes mit den Betriebs- und/oder Geräteparametern fortgesetzt werden, welche zuvor auf der Radiofrequenzmarkierung des Respirationsschlauches abgespeichert worden sind. Betriebsparameter können beispielsweise eine Menge oder Konzentration zu verabreichender Narkotika umfassen.

Die Erfindung wird nun im Folgenden anhand von Figuren und weiteren Ausführungsbeispielen beschrieben.
Figur 1 zeigt ein Ausführungsbeispiel für ein medizinisches System mit zwei medizinischen Arbeitsplätzen;
Figur 2 zeigt ein Ausführungsbeispiel für ein Verfahren zum Zuordnen von Patientendaten zu physiologischen Daten.

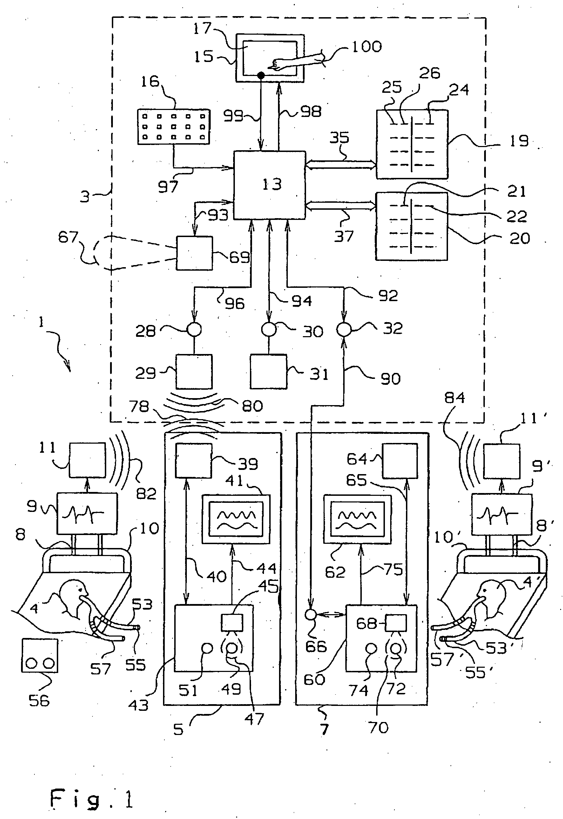

Figur 1 zeigt schematisch ein Ausführungsbeispiel für ein medizinisches System 1. Das medizinische System 1 weist eine Überwachungsstation 3, einen medizinischen Arbeitsplatz 5 und einen medizinischen Arbeitsplatz 7 auf. Die medizinischen Arbeitsplätze 5 und 7 sind jeweils ausgebildet, physiologische Daten eines Patienten 4 zu erfassen. Das medizinische System 1 weist auch einen medizinischen Monitor 9 auf, welcher ausgebildet ist physiologische Daten, insbesondere ein Elektrokardiogramm des Patienten 4 zu erfassen. Der medizinische Monitor 9 ist ausgangsseitig mit einer Schnittstelle 11 verbunden, welche ausgebildet ist, die erfassten Patientendaten schnurlos in Form von schnurlos gesendeten Daten 82 an den medizinischen Arbeitsplatz 5 zu senden. Die Überwachungsstation 3 weist eine zentrale Verarbeitungseinheit 13, eine Anzeigeeinheit 15, welche als Bildwiedergabeeinheit ausgebildet ist und eine berührungsempfindliche Oberfläche 17 auf. Die Überwachungsstation 3 weist auch einen Look-Up-Speicher 19 und einen Look-Up-Speicher 20 auf. Der Look-Up-Speicher 19 und der Look-Up-Speicher 20 sind jeweils zum Vorrätighalten von Datensätzen ausgebildet, von denen die Datensätze 21, 22, 24 und 26 beispielhaft bezeichnet sind. Die in dem Look-Up- Speicher 19 vorrätiggehaltenen Datensätze repräsentieren zusammen eine Look-Up-Tabelle und die in dem Look-Up-Speicher 20 vorrätiggehaltenen Datensätze repräsentieren zusammen eine Look-Up-Tabelle. Die Look-Up-Speicher 19 und 20 sind jeweils als Schreib-Lese-Speicher ausgebildet und können jeweils durch ein dynamisches oder ein statisches RAM (RAM = Random Access Memory) gebildet sein. Die Überwachungsstation 3 weist einen Anschluss 28 zum Anschließen des medizinischen Arbeitsplatzes 5, einen Anschluss 30 zum Anschließen des medizinischen Arbeitsplatzes 7 und einen Anschluss 32 zu einem schnurgebundenen Anschluss des medizinischen Arbeitsplatzes 7 auf. Der Anschluss 28 ist mit einer Schnittstelle 29 zum schnurlosen Empfangen und Senden von Datensignalen verbunden. Der Anschluss 30 ist mit einer Schnittstelle 31 zum schnurlosen Senden und Empfangen eines Datensignals verbunden. Der Anschluss 32 ist zum schnurgebundenen Verbinden mit einem medizinischen Arbeitsplatz ausgebildet und kann beispielsweise durch eine serielle Schnittstelle, insbesondere eine USB-Schnittstelle gebildet sein.

Die zentrale Verarbeitungseinheit 13 ist über einen bidirektionalen Datenbus 35 mit dem Speicher 19 und über einen bidirektionalen Datenbus 37 mit dem Speicher 20 verbunden und ist ausgebildet, wenigstens einen Datensatz aus dem Speicher 19 über den bidirektionalen Datenbus 35 auszulesen oder über diesen einen Datensatz in dem Speicher 19 abzuspeichern. Die zentrale Verarbeitungseinheit 13 kann über den Datenbus 37 einen Datensatz aus dem Speicher 20 auslesen oder über den Datenbus 37 einen Datensatz in dem Speicher 20 abspeichern.

Der medizinische Arbeitsplatz 5 weist eine Schnittstelle 39 zum schnurlosen Empfangen und Senden eines Datensignals auf. In diesem Ausführungsbeispiel kann die Schnittstelle 39 das schnurlos gesendeten Datensignal 82 von dem medizinischen Monitor 9 und der Schnittstelle 11 empfangen. Die Schnittstelle 39 ist über eine Verbindungsleitung 40 mit einer Bildwiedergabeeinheit 41 des medizinischen Arbeitsplatzes 5 verbunden. Die Bildwiedergabeeinheit 41 ist mit einer Respirationsvorrichtung 43 über eine Verbindungsleitung 44 verbunden. Die Respirationsvorrichtung 43 weist einen Anschluss 49 und einen Anschluss 51 auf, welche jeweils zum Anschließen eines Respirationsschlauches vorgesehen sind. Die Respirationsvorrichtung 43 ist ausgebildet, einen Atemgasstrom zum Beatmen eines Patienten, beispielsweise des Patienten 4 zu erzeugen und physiologische Daten, insbesondere betreffend die Respiration, zu erfassen, eine diese repräsentierendes Datensignal zu erzeugen und dieses und über die Verbindungsleitung 44 an die Bildwiedergabeeinheit 41 zu senden. Die Respirationsvorrichtung 43 weist eine Radiofrequenzerfassungsvorrichtung 45 auf. Die Radiofrequenzerfassungsvorrichtung 45 weist einen Erfassungsbereich 47 für eine Radiofrequenzmarkierung auf, welche sich im Bereich des Anschlusses 49 für einen Respirationsschlauch 53 erstreckt. Der Respirationsschlauch 53 ist zum Verbinden mit dem Anschluss 49 ausgebildet und weist im Bereich eines Schlauchendes, welches zum Anschließen mit dem Anschluss 49 vorgesehen ist, eine Radiofrequenzmarkierung 55 mit einer Markierungsinformation auf. Die Radiofrequenzerfassungsvorrichtung 45 ist ausgebildet, die Radiofrequenzmarkierung 55 - beispielsweise nach dem Anschließen des Schlauchendes des Respirationsschlauches 53 in den Anschluss 49 - in dem Erfassungsbereich 47 zu erfassen und ein Markierungssignal zu erzeugen, welches die Markierungsinformation repräsentiert. Die Markierungsinformation kann beispielsweise eine Seriennummer des Respirationsschlauches, oder eine individuelle Identifikationsnummer, welche zum Zuordnen zu einem Patienten vorgesehen ist, repräsentieren. Die Markierungsinformation kann auch persönliche Patientendaten repräsentieren. Dazu kann die Radiofrequenzmarkierung 55 einen Schreib-Lese-Speicher aufweisen, welcher ausgebildet ist, persönliche Patientendaten vorrätig zu halten. Die Respirationsvorrichtung 43 ist über eine Verbindungsleitung 40 mit der Schnittstelle 39 verbunden und kann das mittels der Radiofrequenzerfassungsvorrichtung 45 erzeugte Markierungssignal und/oder ein die physiologischen Respirationsdaten repräsentierendes Datensignal über die Verbindungsleitung 40 an die Schnittstelle 39 senden. Die Schnittstelle 39 kann das über die Verbindungsleitung 40 empfangene, die physiologischen Daten und/oder das schnurlos gesendete Datensignal 82 als schnurlos gesendetes Datensignal 78 an die Überwachungsstation 3 senden. Das schnurlos gesendete Datensignal 78 kann von der Schnittstelle 29 der Überwachungsstation 3 empfangen werden. Der Anschluss 51 für einen Respirationsschlauch ist zum Anschließen des Respirationsschlauches 57 vorgesehen. Der Respirationsschlauch 53 ist in diesem Ausführungsbeispiel für die Inspiration vorgesehen, der Respirationsschlauch 57 ist für die Exspiration.

Der medizinische Arbeitsplatz 7 weist eine Respirationsvorrichtung 60, eine Bildwidergabeeinheit 62, und eine Schnittstelle 64 zum Empfangen eines schnurlos gesendeten Datensignals eines anderen medizinischen Monitors auf. Die Schnittstelle 64 ist über eine Verbindungsleitung 65 mit der Respirationsvorrichtung 60 verbunden. Die Respirationsvorrichtung 60 weist einen Anschluss 66 zum schnurgebundenen Verbinden mit der Überwachungsstation 3 auf. Der Anschluss 66 kann beispielsweise ein USB-Anschluss sein. Die Respirationsvorrichtung 60 weist eine Radiofrequenzerfassungsvorrichtung 68 mit einem Erfassungsbereich 70 für eine Radiofrequenzmarkierung auf, welche sich im Bereich eines Anschlusses 72 für einen Respirationsschlauch erstreckt. Die Respirationsvorrichtung 60 weist auch einen Anschluss 74 für einen weiteren Respirationsschlauch auf. Die Respirationsvorrichtung 60 ist ausgebildet, am Anschluss 72 einen Respirationsluftstrom zur Inspiration und am Anschluss 74 einen Respirationsluftstrom zur Exspiration zu erzeugen. Die Respirationsvorrichtung 60 ist ausgangsseitig über eine Verbindungsleitung 75 mit der Bildwiedergabeeinheit 62 verbunden. Die Respirationsvorrichtung 60 kann physiologische Respirationsdaten erfassen, ein diese repräsentierendes Datensignal erzeugen und dieses über die Verbindungsleitung 75 an die Bildwiedergabeeinheit 62 senden. Die Respirationsvorrichtung 60 kann ein von der Schnittstelle 64 empfangenes, EKG-Datensignal und/oder die erfassten physiologischen Respirationsdaten repräsentierendes Datensignal über den Anschluss 66 an die Überwachungsstation 3 senden.

Der Anschluss 32 der Überwachungsstation 3 ist über eine Verbindungsleitung 90 mit dem Anschluss 66 des medizinischen Arbeitsplatzes 7 trennbar verbunden. Der Anschluss 32 ist über eine Verbindungsleitung 92 mit der zentralen Verarbeitungseinheit 13 verbunden. Der Anschluss 30 ist über eine Verbindungsleitung 94 und der Anschluss 28 ist über eine Verbindungsleitung 96 mit der zentralen Verarbeitungseinheit 13 verbunden. Die zentrale Verarbeitungseinheit ist ausgangsseitig über eine Verbindungsleitung 98 mit der Anzeigeeinheit 15 und über eine Verbindungsleitung 99 eingangsseitig mit der berührungsempfindlichen Oberfläche 17 verbunden.

Die berührungsempfindliche Oberfläche 17 ist ausgebildet, in Abhängigkeit von einem Berühren - beispielsweise durch eine Benutzerhand 100 - ein Benutzerinteraktionssignal zu erzeugen, welches einen Berührungsort des Berührens der berührungsempfindlichen Oberfläche repräsentiert. Die Überwachungsstation 3 weist auch eine Tastatur 16 auf, welche über eine Verbindungsleitung 97 mit der zentralen Verarbeitungseinheit 13 verbunden ist. Die Überwachungsstation 3 weist auch eine Radiofrequenzprogrammiervorrichtung 69 auf, welche ausgebildet ist, in einem Wirkungsbereich 67 eine Radiofrequenzmarkierung zu programmieren und/oder auszulesen, und welche über eine Verbindungsleitung 93 mit der zentralen Verarbeitungseinheit 13 verbunden ist.

Die Funktionsweise und das Zusammenwirken der Komponenten des medizinischen Systems 1 wird nun im Folgenden erläutert:

Die zentrale Verarbeitungseinheit 13 kann über die Tastatur 16 empfangene Patientendaten, beispielsweise Name, Geburtsdatum oder eine Anamnese umfassende Patientendaten erfassen und diese über den bidirektionalen Datenbus 35 in dem Look-Up-Speicher 19 als Patientendatensatz 24 abspeichern. Die zentrale Verarbeitungseinheit 13 kann - beispielsweise in Abhängigkeit eines über die Verbindungsleitung 99 empfangene Benutzerinteraktionssignals - den Patientendatensatz 24 aus dem Look-Up-Speicher 19 auslesen und über die Verbindungsleitung 93 an die Radiofrequenzprogrammiervorrichtung 69 senden. Die Radiofrequenzprogrammiervorrichtung 69 kann den Patientendatensatz auf eine in dem Wirkungsbereich 67 angeordnete Radiofrequenzmarkierung mit einem Schreib-Lese- Speicher übertragen und dort als Markierungsinformation abspeichern. Die Radiofrequenzmarkierung 55 des Respirationsschlauches 53 kann beispielsweise auf diese Weise programmiert worden sein.

Der medizinische Arbeitsplazt 5 ist über die Schnittstelle 39, die Schnittstelle 29 und den Anschluss 28 mit der Überwachungsstation 3, und dort mit der zentralen Verarbeitungseinheit 13 verbunden. Wenn der Respirationsschlauch 53 an den Anschluss 49 der Respirationsvorrichtung 43 angeschlossen wird, kann die Radiofrequenzerfassungsvorrichtung 45 der Respirationsvorrichtung 43 die Radiofrequenzmarkierung 55 in dem Erfassungsbereich 47 erfassen und ein Markierungssignal erzeugen, welches die zuvor in die Radiofrequenzmarkierung 55 einprogrammierten Patientendaten repräsentiert. Die Radiofrequenzerfassungsvorrichtung 45 kann das Markierungssignal, und somit die Patientendaten, über die Verbindungsleitung 40 an die Schnittstelle 39 senden. Die Schnittstelle 39 kann ein schnurlos gesendetes Datensignal 78 erzeugen, welches das Markierungssignal repräsentiert und dieses an die Schnittstelle 29 der Überwachungsstation 3 senden. Das Markierungssignal kann somit über die Schnittstelle 29, den Anschluss 28 und die Verbindungsleitung 96 an die zentrale Verarbeitungseinheit 13 gesendet werden.

Der medizinische Monitor 9, im Folgenden auch EKG-Monitor 9 genannt, kann physiologische Daten des Patienten 4, insbesondere ein Elektrokardiogramm erfassen und ein dieses repräsentierendes EKG-Datensignal erzeugen und dieses über die Schnittstelle 11 an den medizinischen Arbeitsplatz 5 und dort an die Schnittstelle 39 senden. Der medizinische Arbeitsplatz 5 kann über die Verbindungsleitung 40 empfangene physiologische Respirationsdaten und/oder das von dem EKG-Monitor 9 empfangene EKG-Datensignal über die Schnittstelle 39 als schnurlos gesendetes Datensignal 78 an die Überwachungsstation 3 senden. Das schnurlos gesendete Datensignal 78 kann dort von der Schnittstelle 29 schnurlos empfangen und ein dieses entsprechendes Datensignal erzeugt werden, welches über den Anschluss 28, die Verbindungsleitung 96 von der zentralen Verarbeitungseinheit 13 empfangen werden kann. Die zentrale Verarbeitungseinheit 13 kann zeitlich auf das zuvor empfangene Markierungssignal folgende physiologische Daten repräsentierende Datensignal dem Markierungssignal zuordnen. Dazu kann die zentrale Verarbeitungseinheit 13 aus dem Look-Up-Speicher 19 einen dem Markierungssignal entsprechenden Patientendatensatz auslesen und aus dem empfangenen Datensignal einen die physiologischen Daten repräsentierenden Datensatz erzeugen und diesen als Datensatz 26 in dem Look-Up-Speicher 26 abspeichern. Der Datensatz 26 ist in dem Look-Up-Speicher 19 dem Patientendatensatz 24 zugeordnet. Auf diese Weise wird in dem Look-Up-Speicher 19 eine feste Zuordnung von Patientendaten und physiologischen Daten vorrätig gehalten. Die zentrale Verarbeitungseinheit 13 kann den Patientendatensatz 24 und den die physiologischen Daten repräsentierten Datensatz 26 aus dem Look-Up-Speicher 19 über den bidirektionalen Datenbus 35 auslesen und über die Verbindungsleitung 98 an die Anzeigeeinheit 15 senden und mittels der Anzeigeeinheit 15 gemeinsam wiedergeben. Beispielsweise können die physiologischen Daten und die Patientendaten derart gemeinsam wiedergegeben werden, dass die physiologischen Daten eindeutig zu den Patientendaten zugeordnet sind. Beispielsweise können zu Patientendaten zugeordnete physiologische Daten in eine Reihe oder in eine Spalte mittels der Anzeigeeinheit 15 wiedergegeben werden.

Der EKG-Monitor 9 ist mittels einer Haltevorrichtung 8 mit einem Patientenbett 10 verbunden. Das Patientenbett 10 kann im Folgenden beispielsweise zusammen mit dem EKG-Monitor 9 und dem Patienten 4 an einen anderen Ort transportiert werden. Der Respirationsschlauch 53 und der Respirationsschlauch 57 können während eines Transports beispielsweise mit einer mobilen Respirationsvorrichtung 56 verbunden sein. Auf diese Weise kann der Patient 4 auch während des Transports auch weiterhin beatmet werden. Das Patientenbett 10 kann beispielsweise zu dem medizinischen Arbeitsplatz 7 transportiert werden. Der medizinische Arbeitsplatz 7 kann beispielsweise in einem anderen Raum eines Hospitals angeordnet sein. Dargestellt ist das Patientenbett 10 als Patientenbett 10', die Haltevorrichtung als Haltevorrichtung 8', der EKG-Monitor 9 als EKG-Monitor 9', die Schnittstelle 11 als Schnittstelle 11', der Patient 4 als Patient 4', der Respirationsschlauch 53 als Respirationsschlauch 53', der Respirationsschlauch 57 als Respirationsschlauch 57' und die Radiofrequenzmarkierung 55 als Radiofrequenzmarkierung 55', jeweils im Bereich des medizinischen Arbeitsplatzes 7. Wenn der Respirationsschlauch 53' mit dem Anschluss 72 der Respirationsvorrichtung 60 verbunden wird, kann die Radiofrequenzerfassungsvorrichtung 68 die Radiofrequenzmarkierung 55' im Erfassungsbereich 70 erfassen und ein Markierungssignal erzeugen, welches die Markierungsinformation und somit die Patientendaten repräsentiert. Die Respirationsvorrichtung 60 kann das Markierungssignal über den Anschluss 66, die Verbindungsleitung 90, den Anschluss 32 und die Verbindungsleitung 92 an die Überwachungsstation 3 und dort an die zentrale Verarbeitungseinheit 13 senden. Die zentrale Verarbeitungseinheit 13 kann im Folgenden empfangene physiologische Daten repräsentierende Datensignale dem zuvor empfangenen Markierungssignal zuordnen. Der medizinische Arbeitsplatz 7 kann im Folgenden ein von der Schnittstelle 11' empfangenes, schnurlos gesendetes EKG-Datensignal, repräsentierend ein Elektrokardiogramm, über die Schnittstelle 64 empfangen. Die Schnittstelle 64 kann ein dementsprechendes Signal erzeugen und dieses über die Verbindungsleitung 65 an die Respirationsvorrichtung 60 senden.

Die Respirationsvorrichtung 60 kann ein physiologische Daten repräsentierendes Datensignal erzeugen und dieses zusammen mit dem EKG-Datensignal ausgangsseitig über den Anschluss 66 und die Verbindungsleitung 90 an den Anschluss 32 der Überwachungsstation 3 senden. Die zentrale Verarbeitungseinheit 13 kann das die physiologischen EKG-Daten repräsentierende EKG-Datensignal und das die physiologischen Respirationsdaten repräsentierende Signal über die Verbindungsleitung 92 empfangen und dem zuvor empfangenen Markierungssignal zuordnen. Die zentrale Verarbeitungseinheit 13 kann aus dem Look-Up-Speicher 19 einen dem Markierungssignal entsprechenden Patientendatensatz 24 auslesen und einen die entsprechenden physiologischen Daten repräsentierenden Datensatz 25 erzeugen und diesen über die Verbindungsleitung 35 in dem Look-Up-Speicher 19 abspeichern. Dabei ist der Datensatz 25 und der Datensatz 26 dem Patientendatensatz 24 zugeordnet. Der Datensatz 25 kann von der zentralen Verarbeitungseinheit 13, beispielsweise in Abhängigkeit eines über die Verbindungsleitung 99 empfangenen Benutzerinteraktionssignals zusammen mit dem Datensatz 24 und dem Datensatz 26 aus dem Look-Up-Speicher 19 ausgelesen werden und über die Verbindungsleitung 98 an die Anzeigeeinheit 15 gesendet werden und dort jeweils einander zugeordnet wiedergeben werden.

Die zentrale Verarbeitungseinheit 13 kann auch über die Schnittstelle 31 und den Anschluss 30 ein Markierungssignal und ein physiologische Daten repräsentierendes Datensignal empfangen. In diesem Ausführungsbeispiel weist die Überwachungsstation 3 insgesamt drei Anschlüsse zum Anschließen eines medizinischen Monitors auf. Denkbar ist auch eine Überwachungsstation 3 mit einer Vielzahlanschlüssen, welche jeweils zum Anschließen eines medizinischen Monitors ausgebildet sind.

Die Überwachungsstation 3 kann in diesem Ausführungsbeispiel auch mittels der Radiofrequenzprogrammiervorrichtung 69 eine Radiofrequenzmarkierung mit einer Markierungsinformation repräsentierend eine Seriennummer oder eine individuelle Information eines Respirationsschlauches erfassen. Die zentrale Verarbeitungseinheit 13 kann das dementsprechende Markierungssignal über die Verbindungsleitung 93 empfangen und einen entsprechenden Markierungsdatensatz 21 erzeugen und diesen über den bidirektionalen Datenbus 37 in dem Look-Up-Speicher 20 abspeichern. Die zentrale Verarbeitungseinheit 13 kann beispielsweise einen Patientendatensatz 22 erzeugen, und diesen über den bidirektionalen Datenbus 37 in dem Look-Up-Speicher 20 zu dem Markierungs-Datensatz 21 zugeordnet abspeichern. Der Patientendatensatz 22 kann beispielsweise über die Verbindungsleitung 97 oder über die Verbindungsleitung 99 empfangene Patientendaten repräsentieren. Auf diese Weise kann die Überwachungsstation 3 eine eindeutige Zuordnung von Patientendaten und einer Markierungsinformation einer Radiofrequenzmarkierung, beispielsweise der Radiofrequenzmarkierung 55 vorrätig halten. Die zentrale Verarbeitungseinheit 13 kann ein über die Verbindungsleitung 96 oder über die Verbindungsleitung 92 empfangenes Markierungssignal den aus dem Look-Up-Speicher 20 ausgelesenen Markierungs-Datensatz 21 zuordnen. Dazu kann die zentrale Verarbeitungseinheit 13 in dem Look-Up-Speicher 20 vorrätig gehaltene Markierungs-Datensätze auslesen und mit dem zuvor empfangenen Markierungssignal vergleichen. Im Falle einer Übereinstimmung kann die zentrale Verarbeitungseinheit 13 ein entsprechendes Vergleichsergebnis erzeugen und in Abhängigkeit von dem Vergleichsergebnis den in dem Look-Up-Speicher 20 vorrätig gehaltenen, dem entsprechenden Markierungs-Datensatz zugeordneten Patientendatensatz auslesen. Die zentrale Verarbeitungseinheit 13 kann daraufhin den Patientendatensatz über den bidirektionalen Datenbus 35 in dem Look-Up-Speicher 19 abspeichern und im folgenden empfangene physiologische Daten diesem zugeordnet in dem Look-Up-Speicher 19 abspeichern. Die Look-Up-Speicher 19 und 20 können jeweils durch einen gemeinsamen Speicher verwirklicht sein. Beispielsweise kann eine Zuordnung eines Patientendatensatzes, eines Markierungs-Datensatzes und eines Datensatzes repräsentierend physiologische Daten in einer Datenstruktur vorrätig gehalten werden. Die Datenstruktur kann beispielsweise durch eine Datenbank gebildet sein.

Die zentrale Verarbeitungseinheit 13 kann vorteilhaft durch ein Rechenprogramm gesteuert sein.

Die Schnittstellen 11, 29, 31 und 39 können jeweils eine Funk-Schnittstelle, eine Bluetooth-Schnittstelle, eine WLAN-Schnittstelle oder eine Infrarot-Schnittstelle sein.

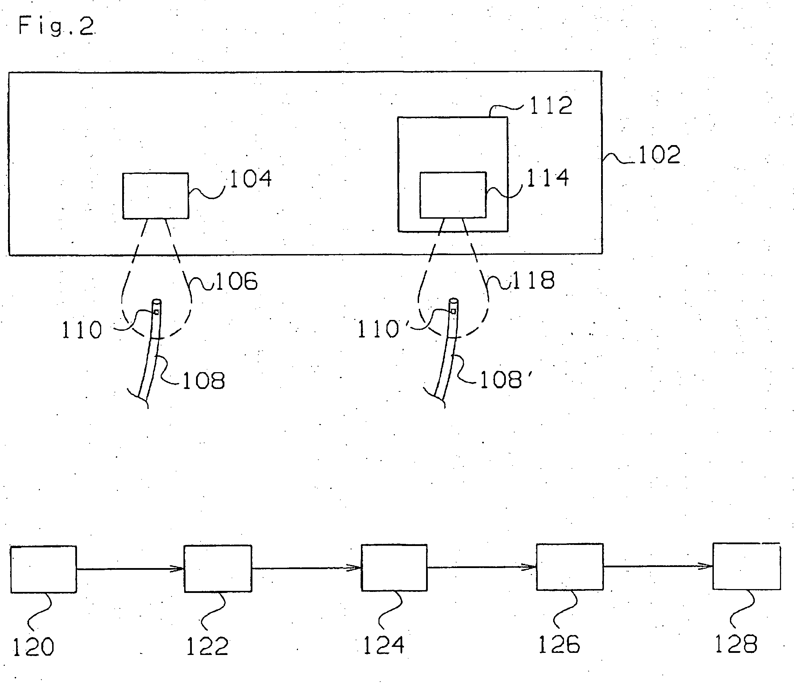

Figur 2 zeigt ein Ausführungsbeispiel für ein Verfahren zum Zuordnen von Patientendaten zu physiologischen Daten. In einem Verfahrensschritt 120 wird eine Radiofrequenzmarkierung 110 eines Respirationsschlauches 108 in einem Erfassungsbereich 106 einer Radiofrequenzerfassungsvorrichtung 104 erfasst und ein Markierungssignal erzeugt, welches die Markierungsinformation der Radiofrequenzmarkierung 110 repräsentiert. In einem weiteren Verfahrensschritt 122 wird die zuvor erfasste Markierungsinformation Patientendaten zugeordnet. In einem Schritt 124 wird die Radiofrequenzmarkierung 110 in einer Position 110' in einem Erfassungsbereich 118 einer Radiofrequenzerfassungsvorrichtung 114 einer Respirationsvorrichtung 112 erfasst und ein die Markierungsinformation repräsentierendes Markierungssignal erzeugt. In einem weiteren Schritt 126 werden physiologische Daten 126 erfasst und der im Schritt 124 erfassten Markierungsinformation zugeordnet. In einem Schritt 128 werden die im Schritt 126 erfassten physiologischen Daten in Abhängigkeit von der im Schritt 126 und im Schritt 122 erfolgten Zuordnung gemeinsam mittels einer Anzeigeeinheit einander zugeordnet wiedergegeben.

Die Radiofrequenzerfassungsvorrichtung 104 und die Respirationsvorrichtung 112 können Bestandteile eines nicht näher dargestellten medizinischen Systems 102 sein, welches von nicht näher dargestellten medizinischen Monitoren physiologische Messdaten erhält.

### Bezugszeichenliste

- 1: medizinisches System
- 3: Überwachungsstation
- 4, 4': Patient
- 5, 7: medizinischer Arbeitsplatz
- 8, 8': Haltevorrichtung
- 10, 10': Patientenbett
- 11, 11': Schnittstelle
- 13: zentrale Verarbeitungseinheit
- 15: Anzeigeeinheit
- 16: Tastatur
- 17: berührungsempfindliche Oberfläche
- 19, 20: Look-Up-Speicher
- 21, 22, 24, 26: Datensatz
- 28, 30, 32: Anschluss
- 29, 31, 39: Schnittstelle
- 35, 37: Datenbus
- 41: Bildwiedergabeeinheit
- 43: Respirationsvorrichtung
- 44: Verbindungsleitung
- 45: Radiofrequenzerfassungsvorrichtung
- 47: Erfassungsbereich
- 49, 51: Anschluss
- 53, 57: Respirationsschlauch
- 55: Radiofrequenzmarkierung
- 60: Respirationsvorrichtung
- 62: Bildwiedergabeeinheit
- 64: Schnittstelle
- 66: Anschluss
- 67: Wirkbereich
- 68: Radiofrequenzerfassungsvorrichtung
- 69: Radiofrequenzprogrammiervorrichtung
- 70: Erfassungsbereich
- 72, 74: Anschluss
- 65, 75, 90, 92: Verbindungsleitung
- 93, 94, 96, 97: Verbindungsleitung
- 98, 99: Verbindungsleitung
- 100: Benutzerhand
- 102: medizinisches System
- 104, 114: Radiofrequenzerfassungsvorrichtung
- 106, 118: Erfassungsbereich
- 108, 108': Respirationsschlauch
- 110, 110': Radiofrequenzmarkierung
- 112: Respirationsvorrichtung
- 120, 122, 124,: Schritt
- 126, 128: Schritt

## Patentansprüche

1. Medizinisches System (1) mit einer Überwachungsstation (3) und mit wenigstens einem medizinischen Arbeitsplatz (5, 7), wobei der wenigstens eine medizinische Arbeitsplatz (5, 7) ausgebildet ist, physiologische Daten eines Patienten zu erfassen, und wobei der wenigstens eine medizinische Arbeitsplatz (5, 7) mit der Überwachungsstation (3) wirkverbunden ist und dazu ausgebildet ist, ein die physiologischen Daten repräsentierendes Datensignal zu erzeugen und an die Überwachungsstation (3) zu senden, wobei die Überwachungsstation (3) ausgebildet ist, das Datensignal zu empfangen, und einen Speicher (19, 20) für wenigstens einen Patientendaten repräsentierenden Patientendatensatz (22, 24) und wenigstens eine Anzeigeeinheit zum Anzeigen der physiologischen Daten (25, 26) und der Patientendaten (22, 24) aufweist, und das medizinische System ausgebildet ist, die Patientendaten (22, 24) und den die physiologischen Daten (25, 26) einander zuzuordnen,
wobei der wenigstens eine medizinische Arbeitsplatz (5, 7) eine Respirationsvorrichtung (43, 60) mit einem Anschluss (49, 72) für einen Respirationsschlauch (53) aufweist und der medizinische Arbeitsplatz (5, 7) eine Radiofrequenzerfassungsvorrichtung (45, 68) aufweist, welche ausgebildet ist, eine Radiofrequenzmarkierung (55) mit einer Markierungsinformation zu erfassen und ein Markierungssignal zu erzeugen, welches die Markierungsinformation repräsentiert, und das medizinische System (1) ausgebildet ist, die physiologischen Daten (25, 26) und die in der erfassten Markierungsinformation enthaltenen Patientendaten (22, 24) in Abhängigkeit des Markierungssignals einander zuzuordnen,
**dadurch gekennzeichnet, dass** die Radiofrequenzerfassungsvorrichtung (45, 68) dazu ausgebildet ist, im Bereich des Anschlusses (49, 72) für einen Respirationsschlauch (53) eine mit dem Respirationsschlauch (53) verbundene Radiofrequenzmarkierung (55) mit der Markierungsinformation zu erfassen und ein Markierungssignal zu erzeugen, welches die Markierungsinformation repräsentiert, wobei die Radiofrequenzmarkierung (55) einen Schreib-Lesespeicher für die Markierungsinformation aufweist und die Markierungsinformation wenigstens die Patientendaten enthält,
und dass der medizinische Arbeitsplatz dazu vorbereitet ist, einen Datensatz mit physiologischen Daten zu erzeugen und diesen, die Patientendaten und einen Datensatz repräsentierend Einstellungs- und Geräteparameter mittels der Radiofrequenzerfassungsvorrichtung auf die Radiofrequenzmarkierung zu übertragen und auf dieser abzuspeichern und den in der Radiofrequenzmarkierung abgespeicherten Datensatz mit den Daten mittels der Radiofrequenzerfassungsvorrichtung auszulesen, wodurch der medizinische Arbeitsplatz einen weiteren Betrieb in Abhängigkeit von den ausgelesenen Daten durchführen oder fortsetzten kann.

2. Medizinisches System nach einem der vorhergehenden Ansprüche,
**dadurch gekennzeichnet,**
**dass** wenigstens ein medizinischer Arbeitsplatz (5) drahtlos mit der Überwachungsstation (3) verbunden ist.

3. Medizinisches System nach einem der vorhergehenden Ansprüche,
**dadurch gekennzeichnet,**
**dass** wenigstens ein medizinischer Arbeitsplatz (7) drahtgebunden mit der Überwachungsstation (3) verbunden ist.

4. Verfahren zum Zuordnen von Patientendaten zu physiologischen Daten in einem medizinischen System mit einer Überwachungsstation (3) und mit wenigstens einem medizinischen Arbeitsplatz (5, 7), bei dem physiologische Daten eines Patienten von dem medizinischen Arbeitsplatz (5, 7) erfasst und von diesem ein die physiologischen Daten repräsentierendes Datensignal erzeugt und an die Überwachungsstation (3) gesendet wird, die das Datensignal empfängt, wobei der medizinische Arbeitsplatz (5, 7) einen Speicher (19, 20) für wenigstens einen Patientendaten repräsentierenden Patientendatensatz (22, 24) und wenigstens eine Anzeigeeinheit zum Anzeigen der physiologischen Daten (25, 26) und der Patientendaten (22, 24) aufweist, wobei die Patientendaten (22, 24) und die physiologischen Daten (25, 26) von dem medizinischen Arbeitsplatz (5, 7) einander zugeordnet werden,
wobei der wenigstens eine medizinische Arbeitsplatz (5, 7) eine Respirationsvorrichtung (43, 60) mit einem Anschluss (49, 72) für einen Respirationsschlauch (53) aufweist und der medizinische Arbeitsplatz (5, 7) mit einer Radiofrequenzerfassungsvorrichtung (45, 68) eine Radiofrequenzmarkierung (55) mit einer Markierungsinformation erfasst und ein Markierungssignal erzeugt, das die Markierungsinformation repräsentiert, wobei das einander Zuordnen der physiologischen Daten (25, 26) und der in der in der erfassten Markierungsinformation enthaltenen Patientendaten (22, 24) in Abhängigkeit von dem Markierungssignal erfolgt,
**dadurch gekennzeichnet, dass** die Radiofrequenzerfassungsvorrichtung im Bereich des Anschlusses für einen Respirationsschlauch eine mit dem Respirationssschlauch (53) verbundene Radiofrequenzmarkierung (55) mit der Markierungsinformation erfasst und ein Markierungssignal erzeugt, das die Markierungsinformation repräsentiert, wobei die Radiofrequenzmarkierung (55) einen Schreib-Lesespeicher für die Markierungsinformation aufweist und die Markierungsinformation wenigstens die Patientendaten repräsentiert,
und dass der medizinische Arbeitsplatz (5, 7) einen Datensatz mit physiologischen Daten erzeugt und diesen, die Patientendaten und einen Datensatz repräsentierend Einstellungs- oder Geräteparameter mittels der Radiofrequenzerfassungsvorrichtung auf die Radiofrequenzmarkierung überträgt und auf dieser abspeichert und dem in der Radiofrequenzmarkierung abgespeicherten Datensatz mit den Daten mittels der Radiofrequenzerfassungsvorrichtung ausliest, wodurch der medizinische Arbeitsplatz einen weiteren Betrieb in Abhängigkeit von den ausgelesenen Daten durchführen oder fortsetzen kann und ein Betrieb an einem anderen medizinischen Arbeitsplatz mit den zuvor auf der Radiofrequenzmarkierung (55) des Respirationsschlauches (53)gespeicherten Einstellungs- oder Geräteparametern fortgesetzt werden kann.

5. Verfahren nach Anspruch 4,
**dadurch gekennzeichnet,**
**dass** die Markierungsinformation Einstellungs- oder Geräteparameter enthält, welche individuell für einen Patienten erstellt worden sind.

## Claims

1. A medical system (1) having a monitoring station (3) and having at least one medical workstation (5, 7), wherein the at least one medical workstation (5, 7) is designed to acquire physiological data of a patient, and wherein the at least one medical workstation (5, 7) is operatively connected to the monitoring station (3) and is designed to generate a data signal representing the physiological data and to send it to the monitoring station (3), wherein the monitoring station (3) is designed to receive the data signal and has a memory (19, 20) for at least one patient data record (22, 24), representing patient data, and at least one display unit to display the physiological data (25, 26) and the patient data (22, 24), and the medical system is designed to associate the patient data (22, 24) and the physiological data (25, 26) with each other, wherein the at least one medical workstation (5, 7) has a respiratory device (43, 60) with a connection (49, 72) for a respiratory hose (53), and the medical workstation (5, 7) has a radio-frequency-detection device (45, 68) which is designed to detect a radio-frequency marker (55) with marker information and to generate a marker signal which represents the marker information, and the medical system (1) is designed to associate the physiological data (25, 26) and the patient data (22, 24) contained in the detected marker information with each other as a function of the marker signal,
**characterised in that** the radio-frequency-detection device (45, 68) is designed to detect in the region of the connection (49, 72) for a respiratory hose (53) a radio-frequency marker (55), connected with the respiratory hose (53), with the marker information and to generate a marker signal which represents the marker information, wherein the radio-frequency marker (55) has a read-write memory for the marker information, and the marker information contains at least the patient data,
and **in that** the medical workstation is set up to generate a data record with physiological data and to transmit and store this, the patient data and a data record representing adjustment and equipment parameters by means of the radio-frequency-detection device to and on the radio-frequency marker and to read out the data record, stored in the radio-frequency marker, with the data by means of the radio-frequency-detection device, whereby the medical workstation can carry out or continue a further operation as a function of the data that have been read out.

2. A medical system according to one of the preceding claims,
**characterised in that**
at least one medical workstation (5) is connected to the monitoring station (3) wirelessly.

3. A medical system according to one of the preceding claims,
**characterised in that**
at least one medical workstation (7) is connected to the monitoring station (3) by wire.

4. A method for associating patient data with physiological data in a medical system having a monitoring station (3) and having at least one medical workstation (5, 7), in which physiological data of a patient are acquired by the medical workstation (5, 7), and from this a data signal representing the physiological data is generated and sent to the monitoring station (3) that receives the data signal, wherein the medical workstation (5, 7) has a memory (19, 20) for at least one patient data record (22, 24), representing patient data, and at least one display unit to display the physiological data (25, 26) and the patient data (22, 24), wherein the patient data (22, 24) and the physiological data (25, 26) from the medical work station (5, 7) are associated with each other,
wherein the at least one medical workstation (5, 7) has a respiratory device (43, 60) with a connection (49, 72) for a respiratory hose (53), and the medical workstation (5, 7) with a radio-frequency-detection device (45, 68) detects a radio-frequency marker (55) with marker information and generates a marker signal which represents the marker information,
wherein the physiological data (25, 26) and the patient data (22, 24) contained in the detected marker information are associated with each other as a function of the marker signal,
**characterised in that** the radio-frequency-detection device detects in the region of the connection for a respiratory hose a radio-frequency marker (55), connected with the respiratory hose (53), with the marker information and generates a marker signal which represents the marker information, wherein the radio-frequency marker (55) has a read-write memory for the marker information, and the marker information represents at least the patient data,
and **in that** the medical workstation (5, 7) generates a data record with physiological data and transmits and stores this, the patient data and a data record representing adjustment or equipment parameters by means of the radio-frequency-detection device to and on the radio-frequency marker and reads out the data record, stored in the radio-frequency marker, with the data by means of the radio-frequency-detection device, whereby the medical workstation can carry out or continue a further operation as a function of the data that have been read out, and an operation can be continued at a different medical workstation with the adjustment or equipment parameters previously stored on the radio-frequency marker (55) of the respiratory hose (53).

5. A method according to claim 4,
**characterised in that**
the marker information contains adjustment or equipment parameters that have been generated individually for a patient.

## Revendications

1. Système médical (1) comprenant une station de surveillance (3) et au moins un poste de travail médical (5, 7), ledit au moins un poste de travail médical (5, 7) étant réalisé pour détecter des données physiologiques d'un patient et ledit au moins un poste de travail médical (5, 7) étant en liaison fonctionnelle avec la station de surveillance (3) et étant réalisé pour générer et envoyer à la station de surveillance (3) un signal de données représentant les données physiologiques, la station de surveillance (3) étant réalisée pour recevoir le signal de données et présentant une mémoire (19, 20) pour au moins un jeu de données de patient (22, 24) représentant des données de patient et au moins une unité d'affichage pour afficher les données physiologiques (25, 26) et les données de patient (22, 24), et le système médical étant réalisé pour associer entre elles les données de patient (22, 24) et les données physiologiques (25, 26),
ledit au moins un poste de travail médical (5, 7) présentant un dispositif de respiration (43, 60) avec un raccord (49, 72) pour un tuyau respiratoire (53) et le poste de travail médical (5, 7) présentant un dispositif de détection de radiofréquence (45, 68) qui est réalisé pour détecter un marquage par radiofréquence (55) comprenant une information de marquage et pour générer un signal de marquage qui représente l'information de marquage, et le système médical (1) étant réalisé pour associer entre elles les données physiologiques (25, 26) et les données de patient (22, 24) contenues dans l'information de marquage détectée en fonction du signal de marquage,
**caractérisé en ce que** le dispositif de détection de radiofréquence (45, 68) est réalisé pour détecter, dans la zone du raccord (49, 72) pour un tuyau respiratoire (53), un marquage par radiofréquence (55) comprenant l'information de marquage lié au tuyau respiratoire (53) et pour générer un signal de marquage qui représente l'information de marquage, le marquage par radiofréquence (55) présentant une mémoire d'écriture-lecture pour l'information de marquage et l'information de marquage contenant au moins les données de patient,
et **en ce que** le poste de travail médical est préparé pour générer un jeu de données avec des données physiologiques et pour transmettre et enregistrer celui-ci, les données de patient et un jeu de données représentant des paramètres de réglage et d'appareil sur le marquage par radiofréquence au moyen du dispositif de détection de radiofréquence et pour lire le jeu de données comprenant les données enregistré dans le marquage par radiofréquence au moyen du dispositif de détection de radiofréquence, de sorte que le poste de travail médical peut fonctionner ou continuer à fonctionner par la suite en fonction des données lues.

2. Système médical selon l'une des revendications précédentes,
**caractérisé en ce**
**qu'**au moins un poste de travail médical (5) est relié sans fil à la station de surveillance (3).

3. Système médical selon l'une des revendications précédentes,
**caractérisé en ce**
**qu'**au moins un poste de travail médical (7) est relié par fil à la station de surveillance (3).

4. Procédé pour associer des données de patient à des données physiologiques dans un système médical comprenant une station de surveillance (3) et au moins un poste de travail médical (5, 7), selon lequel des données physiologiques d'un patient sont détectées par le poste de travail médical (5, 7), lequel génère un signal de données représentant les données physiologiques et l'envoie à la station de surveillance (3), laquelle reçoit le signal de données, le poste de travail médical (5, 7) présentant une mémoire (19, 20) pour au moins un jeu de données de patient (22, 24) représentant des données de patient et au moins une unité d'affichage pour afficher les données physiologiques (25, 26) et les données de patient (22, 24), les données de patient (22, 24) et les données physiologiques (25, 26) étant associées entre elles par le poste de travail médical (5, 7),
ledit au moins un poste de travail médical (5, 7) présentant un dispositif de respiration (43, 60) avec un raccord (49, 72) pour un tuyau respiratoire (53) et le poste de travail médical (5, 7) détectant un marquage par radiofréquence (55) comprenant une information de marquage au moyen d'un dispositif de détection de radiofréquence (45, 68) et générant un signal de marquage qui représente l'information de marquage, l'association entre elles des données physiologiques (25, 26) et des données de patient (22, 24) contenues dans l'information de marquage détectée étant effectuée en fonction du signal de marquage,
**caractérisé en ce que** le dispositif de détection de radiofréquence détecte, dans la zone du raccord pour un tuyau respiratoire, un marquage par radiofréquence (55) comprenant l'information de marquage lié au tuyau respiratoire (53) et génère un signal de marquage qui représente l'information de marquage, le marquage par radiofréquence (55) présentant une mémoire d'écriture-lecture pour l'information de marquage et l'information de marquage représentant au moins les données de patient,
et **en ce que** le poste de travail médical (5, 7) génère un jeu de données avec des données physiologiques et transmet et enregistre celui-ci, les données de patient et un jeu de données représentant des paramètres de réglage ou d'appareil sur le marquage par radiofréquence au moyen du dispositif de détection de radiofréquence et lit le jeu de données contenant les données enregistré dans le marquage par radiofréquence au moyen du dispositif de détection de radiofréquence, de sorte que le poste de travail médical peut fonctionner ou continuer à fonctionner par la suite en fonction des données lues et qu'un fonctionnement peut être poursuivi sur un autre poste de travail médical avec les paramètres de réglage ou d'appareil enregistrés préalablement sur le marquage par radiofréquence (55) du tuyau respiratoire (53).

5. Procédé selon la revendication 4,
**caractérisé en ce**
**que** l'information de marquage contient des paramètres de réglage ou d'appareil qui ont été créés individuellement pour un patient.
